# EUROPEAN PATENT SPECIFICATION

(11) **EP 2 329 268 B1**
(45) Date of publication and mention of the grant of the patent: **06.11.2013**
(21) Application number: 09783500.3
(22) Date of filing: 28.09.2009
(51) Int. Cl.: G01N 33/542, G01N 33/566

(54) **METHODS AND COMPOUNDS FOR TESTING BINDING OF A LIGAND TO A G PROTEIN-COUPLED RECEPTOR**
VERFAHREN UND VERBINDUNGEN ZUM TESTEN DER BINDUNG EINES LIGANDEN AN EINEN G-PROTEIN-GEKOPPELTEN REZEPTOR
MÉTHODES ET COMPOSÉS POUR UN ESSAI DE LIAISON D'UN LIGAND À UN RÉCEPTEUR COUPLÉ À LA PROTÉINE G

(30) Priority: 30.09.2008 GB 0817861
(43) Date of publication of application: 08.06.2011
(73) Proprietor: GE Healthcare UK Limited, Buckinghamshire HP7 9NA (GB)
(72) Inventor: SANTOS, Albert, Francis, Cardiff South Glamorgan CF14 7YT (GB)
(74) Representative: Bryan, Ian Bennett
(86) International application number: PCT/EP2009/062546
(87) International publication number: WO 2010/037718

(56) References cited:
- WO-A1-2006/035208
- WO-A2-2005/031309
- VERKAAR F ET AL: "G protein-independent cell-based assays for drug discovery on seven-transmembrane receptors" BIOTECHNOLOGY ANNUAL REVIEW 2008 NL, vol. 14, 2008, pages 253-274, XP008116252

## Description

### Field of the Invention

The present invention relates to the field of cell biology, molecular biology and drug screening. In particular, the invention relates to G Protein-Coupled Receptors (GPCRs) and to compounds and methods for testing the binding of ligands to GPCRs.

### Background to the Invention

### G Protein-Coupled Receptors

G protein-coupled receptors (GPCRs), also known as seven transmembrane domain receptors, 7TM receptors and G protein-linked receptors (GPLR), comprise a large protein family of trans-membrane receptors that bind molecules outside the cell and activate signal transduction pathways and, ultimately, cellular responses. GPCRs are found only in eukaryotes, including yeast, plants, flagellate protozoa, animals. The ligands that bind and activate these receptors include light-sensitive compounds, odours, pheromones, hormones, neurotransmitters, and drugs, and vary in size from small molecules such as peptides to large proteins. As GPCRs are involved in many diseases, they are the target of many modern medicines. Drugs active at GPCRs have therapeutic benefit across a broad spectrum of human diseases as diverse as pain, cognitive dysfunction, hypertension, peptic ulcers, rhinitis, asthma, inflammation, obesity and cancer, as well as cardiovascular, metabolic, gastrointestinal, visual and neurodegenerative diseases. Of the total clinically marketed drugs, greater than 30% are modulators of GPCR function, representing 9% of global pharmaceutical sales, making GPCRs the most successful of any target class in terms of drug discovery. GPCRs represent the single most important class of drug targets and significant targets in drug discovery. Indeed, 20% of the top fifty best selling drugs act at GPCRs, which equates to approximately $25 billion in terms of pharmaceutical sales per annum. However, current drugs exhibit their activity at less than 10% of known GPCRs, implying that there is large potential for further discovery. Indeed, the DNA sequences of a large number of GPCRs can be found in public databases, among other sources, leading to identification of putative so-called "orphan receptors". These orphan receptors are defined as those not acted upon by a known endogenous ligand. One challenge for the drug development industry is to associate the many orphan GPCRs with disease to potentially identify novel pharmaceutical agents of the future.

GPCRs are closely associated with heterotrimeric G-proteins that are bound to the inner face of the plasma membrane. G-proteins are key molecular components in the intracellular signal transduction following ligand binding to the extracellular domain of a GPCR. The G-protein subunits historically are designated α, β, and γ, and their classification is largely based on the identity of their distinct α subunits, and the nature of the subsequent transduction event (Table 1). Further classification of G-proteins has come from cDNA sequence homology analysis. G-proteins bind either guanosine diphosphate (GDP) or guanosine triphosphate (GTP), and possess highly homologous guanine nucleotide binding domains and distinct domains for interactions with receptors and effectors.

When the GPCR "system" is inactive (i.e. in the absence of ligand), GDP is bound to the Gα subunit. An agonist-receptor complex induces conformational changes in the GPCR/G-protein complex, which facilitates preferential binding of GTP to the Gα subunit, in part by promoting the dissociation of bound GDP. This so-called "guanyl nucleotide exchange" is critical. Binding of GTP activates the Gα subunit, leading to dissociation through space from the Gβγ dimer. The Gα and Gβγ subunits are then able to subsequently activate, either independently or in parallel, downstream effectors such as adenylate cylase, calcium, phospholipase activity or other ions.

Termination of signal transduction results from hydrolysis of bound GTP to GDP by a GTPase enzyme that is intrinsic to the α subunit, leading to re-association of α and βγ subunits. Thus, G- proteins serve as regulated molecular switches capable of eliciting bifurcating signals through α and βγ subunit effects. The switch is turned on by the receptor and it turns itself off within a few seconds, a time sufficient for considerable amplification of signal transduction

**Table 1. Examples of the relationship of G Protein-Coupled Receptors and Signalling Pathways**

| **G- Protein Subunit Regulation** | **Effectors / Signalling Pathways** |
|---|---|
| αs | Adenylate cyclase (cAMP) ↑ |
| αi | Adenylate cyclase (cAMP) ↓ |
| αo | Ca²⁺↓ |
| αq | Phospholipase C (IP₃) ↑ |
| α13 | Na⁺/H⁺ exchange ↑ |
| αt | cGMP-phosphodiesterase (vision) ↑ |
| αolf | Adenylate cyclase (cAMP) ↑ |
| βγ | K⁺ channels |
| βγ | Adenylate cyclase (cAMP) ↑or↓ |
| βγ | Phospholipase C (IP₃) |

### Structure of GPCRs

GPCRs are integral hydrophobic membrane proteins that span the plasma membrane in seven α-helical segments. The extracellular binding site for small GPCR-active ligands is a pocket within the bundle of membrane-spanning helices, but a substantial extracellular domain is important for the binding of the negatively charged ligands. GPCRs are activated by an external signal in the form of a ligand or other signal mediator. This creates a conformational change in the receptor, causing activation of a G-protein. Further effect depends on the type of G-protein. The receptors interact with G proteins at their cytoplasmic face, and it has been possible to define specific regions within GPCR structures that are responsible for regulation of and selectivity among the different G-proteins.

In order to study GPCR activation, a variety of functional biochemical and cellular assay methodologies are typically used. Examples of functional assay systems for monitoring GPCR activation include the intracellular measurement of the GPCR effector targets, cAMP, cGMP and IP₃. A number of homogeneous assay methodologies such as Scintillation Proximity Assay (SPA), Fluorescence Polarization (FP) and Enzyme Fragment Complementation (EFC) have been successfully used for the measurement of these agents. Furthermore, as described above, ligand-induced stimulation of GPCRs results in the exchange of GDP for GTP, and this event can be monitored by the binding of radiolabelled [³⁵S] GTPγS, for example.

In the process of drug discovery and lead optimisation, there is a requirement for faster, more effective, less expensive and especially information-rich screening assays that provide simultaneous information on various compound characteristics and their affects on various cellular pathways (i.e. efficacy, specificity, toxicity and drug metabolism). Such assays would allow drug discovery to identify drug candidates capable of activating or blocking GPCR signalling. Thus, for drug discovery, there is a need to quickly and inexpensively screen large numbers of chemical compounds to identify new drug candidates, including receptor agonists, inverse agonists and antagonists as well as inhibitors of GPCRs and GPCR-dependent pathways. These chemical compounds may be collected in large libraries, sometimes exceeding one million distinct compounds.

Traditional biochemical approaches for assaying GPCRs have relied upon measurements of ligand binding, for example with filter binding assays (heterogeneous) or with SPA (homogeneous). Although such assays are inexpensive to carry out, development time can be lengthy in some cases.

A major problem is that the development of a traditional assay requires specific reagents for every target of interest including purified protein for the target against which the screen is to be run. Often it is difficult to express the protein of interest and/or to obtain a sufficient quantity of the protein in pure form.

Although binding assays are the gold standard for pharmacology and studies of structure-activity relationships (SAR), it is not usually possible to perform target validation with binding assays. The increased numbers of drug targets identified by genomic approaches has driven the development of gene-to-screen approaches to interrogate poorly- defined targets, many of which rely on cellular assay systems. Speculative targets are most easily screened in a format in which the target is expressed and regulated in the biological context of a cell, in which all of the necessary components are pre-assembled and regulated. Cell-based assays are also critical for assessing the mechanism of action of new biological targets and biological activity of chemical compounds. In particular, there is a need to "de-orphanise" those GPCRs for which natural activating ligand has not been identified. Various approaches to "de-orphanisation" have been adopted including array-screening against families of known ligands. Current cell-based assays for GPCRs include measures of pathway activation (Ca²⁺ release, cAMP generation or transcriptional activity); measurements of protein trafficking by tagging GPCRs and down stream elements with GFP; and direct measures of interactions between proteins using Förster resonance energy transfer (FRET), bioluminescence resonance energy transfer (BRET) or yeast two-hybrid approaches.

### Definitions

The meaning of the terms "agonist", "inverse agonist" and "antagonist" as used herein are based on the definition given in:
"Definitions, GlaxoWellcome Pharmacology Guide", at
http://www2.uah.es/farmamol/Public/pharmacol_guide/chap2c.html

An "agonist" is a compound or drug which binds to a receptor and activates it, producing a pharmacological response (e.g. contraction, relaxation, secretion, enzyme activation, etc.).

An "inverse agonist" is a compound or drug which produces an effect opposite to that of an agonist, yet acts at the same receptor. The best established examples act at the benzodiazepine receptor. Such compounds have also been described as "negative antagonists", or as having "negative efficacy".

An "antagonist" is a compound or drug which attenuates the effect of an agonist. It may be competitive, i.e. it binds reversibly to a region of the receptor in common with an agonist but occupies the site without activating the effector mechanism. The effects of a competitive antagonist may be overcome by increasing the concentration of agonist, thereby shifting the equilibrium and increasing the proportion of receptors which the agonist occupies.
Alternatively, antagonists may be non-competitive, where no amount of agonist can completely overcome the inhibition once it has been established. Non-competitive antagonists may bind covalently to the agonist binding site ("competitive irreversible antagonists"), in which case there is a period before the covalent bond forms during which competing ligands can prevent the inhibition. Other types of non-competitive antagonists act allosterically at a different site on the receptor or an associated ion channel.

The term "testing" as used herein, shall include but not be limited to, detecting, measuring and/or quantifying.

### Cell Based Assays

Traditional cell-based assays for GPCRs often rely upon measurements of intracellular calcium flux. Calcium release from intracellular stores is stimulated by specific classes of GPCRs upon their activation; in particular those GPCRs that couple to a G-protein known as Gq (or Gq/11). Fluorescent and luminescent assays of calcium release have been generated by loading cells with dyes that act as calcium indicators. Fluorescent calcium indicators such as Fura-2, Indole-1, Fluo-3 and calcium green have been widely used for measurement of intracellular calcium measurement. Such indicators and associated instrumentation such as FLIPR (Molecular Devices, Sunnyvale, California, USA) are well established tools. Luminescent assays of calcium transients can be also carried out, by the introduction of aequorin into cells, usually with genetic engineering techniques. Aequorin emits blue light in the presence of calcium, and the rate of photon emission is proportional to the free calcium concentration within a specific range. Cells expressing the GPCR of interest are loaded first with coelenterazine to activate the aequorin, and then the compounds to be tested are added to the cells and the results measured with a luminometer. To extend these assays to non-Gq-coupled receptors, various engineering strategies have been used, including the use of a promiscuous Gα protein construct such as Gα16 that is capable of coupling a wide range of GPCRs to phospholipase C (PLC) activity and calcium mobilisation.

Adenylyl cyclases are a family of membrane-bound enzymes that are linked to G protein-coupled receptors and influence the regulation of cell function in virtually all cells. cAMP is synthesized by adenylyl cyclase in response to activation of many receptors; stimulation is mediated by Gs, and inhibition by one or more closely related G proteins termed Gi's. There exist at least ten tissue-specific adenylyl cyclases each with its unique pattern of regulatory responses. Several adenylyl cyclase isozymes are inhibited by the G protein βγ subunits, which allow activation of G proteins other than Gs to inhibit cyclase activity. Other isozymes are stimulated by Gβγ subunits, but this stimulation is dependent upon concurrent stimulation by the α subunit of Gs. Still other isozymes are stimulated by Ca²⁺ or Ca²⁺⁻calmodulin complexes. Finally, each of the isozymes has its own pattern of enhancement or attenuation by phosphorylation or other regulatory influences, providing a broad array of regulatory features to the target cells where these isoforms are expressed.

cAMP is a ubiquitous second messenger and functions as one of many signalling molecules enabling cells to respond to external signals. cAMP assays are used to monitor cellular responses to either Gs or Gi-coupled receptor activation. Typically, with cAMP, the binding of a hormone, agonist or neuromodulator to its receptor is followed by activation or inhibition of a G-protein which, in turn, activates adenylate cyclase, evoking the generation of cAMP from ATP. The activation of protein kinase A by cAMP results in the phosphorylation of specific substrates, which include enzymes, ion channels and transcription factors. Because cAMP can activate a cascade of reactions, the involvement of cAMP greatly amplifies the cellular response to a variety of drugs and hormonal stimuli. Therefore, measurement of intracellular cAMP generation has become an established means of screening for antagonists and agonists of receptors linked to adenylate cyclase via either inhibitory or stimulatory G-proteins.

Fluorescent dyes, and fluorescent proteins such as GFP, YFP, BFP and CFP have also been used as cellular sensors of cAMP and calcium. The first protein indicator for cAMP consisted of the cyclic AMP-dependent protein kinase, PKA, in which the catalytic and regulatory subunits were labelled with fluorescein and rhodamine, respectively, so that cAMP-induced dissociation of the subunits disrupted FRET between the dyes. Replacement of the dyes by GFP and BFP made this system genetically encodable and eliminated the need for *in vitro* dye conjugation and microinjection.

Transcriptional reporter assays provide a measurement of pathway activation or inhibition in response to an agonist/antagonist, and have been used extensively in GPCR studies. Reporter-gene assays couple the biological activity of a receptor to the expression of a readily detectable enzyme or protein reporter. Synthetic repeats of a particular response element can be inserted upstream of the reporter gene to regulate its expression in response to signalling molecules generated by activation of a specific pathway in a live cell. Such drug screening systems have been developed with a variety of enzymatic and fluorescent reporters, including β-galactosidase, luciferase, alkaline phosphatase, green fluorescent protein (GFP), β-lactamase) and others. Transcription reporter assays are highly sensitive screening tools; however, they do not provide information on the mechanism of action of the compound. The latter would enable mapping of the components of the pathway, leading to transcription, or enable studies of the individual steps within signalling cascades.

High content screening (HCS) is an approach that, in one format, relies upon imaging of cells to detect the sub-cellular location and trafficking of proteins in response to stimuli or inhibitors of cellular processes.

Kimple et al. (2006) Combinatorial Chemistry & High Throughput Screening describes established and emerging fluorescence-based assays for G-protein function.

Fluorescent probes can be used in HCS. For example, GTP has been labelled with the fluorescent dye, BODIPY, and used to study the on/off-rates of GTP hydrolysis by G-proteins.

Fluorescein-labelled myristoylated Gαi has also been used as a ligand that binds Gβγ in order to study the association and dissociation of G-protein subunits. GFP has been used to analyse key signalling events within cells. By fusing in-frame a cDNA for GFP to a cDNA coding for a protein of interest, it is possible to examine the function and fate of the resulting chimera in living cells. This strategy has now been applied to nearly all known elements of G-protein coupled pathways including the receptors themselves, G-protein subunits such as Gα; β-arrestin, RGS proteins, protein kinase C and other intracellular components of G-protein-coupled pathways. For example, GPCRs have been tagged with GFP in order to monitor receptor internalization. A fusion protein comprising GFP-β-arrestin has been shown to co-localise with thyrotropin-releasing hormone receptor 1 in response to agonist. GFP has been introduced internally to G-proteins, creating a Gα/GFP chimera, which has been shown to translocate to the cell membrane upon GPCR activation. GFP tagging has also been used to monitor intracellular signalling events. GFP tagged RGS proteins were selectively recruited to the plasma membrane by G-proteins and their receptors. GFP-tagged protein kinase C (PKC), which is activated by the release of diacylglycerol from cell membranes, has been used to monitor translocation of the kinase in response to cell signalling.
In addition, GFP-tagged connexion has been used to monitor intracellular calcium flux.

US 5891646 and US6110693 (Norak Bioscience) describe GFP- tagged β-arrestin and this has been used to monitor GPCR activation by imaging the subcellular redistribution of β-arrestin in response to agonist. However, the signal generated from GFP translocation methods is very low and is prone to instrument interference which can result in poor assay results

PCT WO 2005/121755 and Leifert et al. (2006) Anal. Biochem., 355, 201-212 describe a cell-free GPCR and ligand assay using chemically-generated fluor molecules in a FRET assay.

An homogeneous GTP binding assay for G protein-coupled receptors based on time resolved FRET has previously been described (Frang, H., et al. GTP binding assay for GPCRs based on TR-FRET, Poster PO 8123, Ninth Annual Society for Biomolecular Screening, Portland, Oregon, 21-25 September 2003). In this assay, a biotinylated BioKey® peptide is employed that recognizes only the GTP bound form of the Gα subunit. The biotinylated peptide enables binding of streptavidin-europium in close proximity to an acceptor-labelled GTP, which is also bound to the Gα subunit. FRET occurs as a result of interaction between the streptavidin-europium (donor) and the fluorescently-labelled GTP analogue (Alexa647-GTP).

WO 2006/035207 (GE Healthcare UK Limited) describes fluorescent cyanine dye labeled nucleotide analogues in which the cynanine dye is coupled to the γ-phosphate group of a nucleoside triphosphate. These GTPase resistant analogues can be used in an homogenous FRET-based assay to measure the binding of guanine nucleotides to GPCR polypeptides, or alternatively, to measure the effect of an exogenous ligand on GPCR binding. Further uses of similar GTPase resistant GTP analogues in GPCR binding assays are disclosed in WO2006/035208 (GE Healthcare UK Limited).

While a variety of fluorescent dye-nucleotide conjugates are available, the selection of a particular fluorescent label for use in a protein binding assay can be problematic, since the electronic and spatial requirements of the binding site of the protein of interest are difficult to predict *a priori.* There is therefore, still a requirement for new GTP analogues that may be used for quantitating G-proteins and for studying the kinetics of agonist induced guanine nucleotide exchange in *in vitro* assays and in cellular systems.

### Enzyme Complementation

*β*-galactosidase (*β*-gal) is a tetrameric enzyme with a MW of 464,000. Each identical subunit contains 1021 amino acids, encoded in *E*. *coli* by the Z gene of the lac operon promoter. In *E*. *coli*, intracistronic *β* galactosidase (*β*-gal) complementation is a naturally occurring phenomenon, and involves α and ω Complementation of amino and carboxyl-terminal domains of the *lac* Z enzyme.

Ullmann *et al.* (Ullmann A, Perrin D, Jacob F, Monod J (1965). J Mol Biol., 12, 918-923) described the complementation of *β* -gal in *E*. *coli.* A peptide was found (Peptide "ω") that was present in extracts of various mutants ("ω donors") of the (Z) promoter gene. The ω peptide complemented *β* -gal activity when added to extracts containing a β-gal (-ve) mutant (" ω acceptor"). The w enzyme acceptor peptide (EA) has since been found to lack residues 11-41, and is frequently referred to as the M15 protein, since it is a product of the lac Z M15 allele. Sucrose density assessments suggested a MW of 30,000 to 40,000 for the ω peptide and in an operator-distal segment of the z gene. A following publication by Ullmann *et al.* (Ullmann A, Jacob F, Monod J, 1967 J Mol Biol., 24, 339-343) described how extracts from various *β* -gal-negative mutants were screened for their capacity to complement with extracts of partial deletions of the operator-proximal segment ("α") of the z gene. Together, the operator-distal (ω) and the operator-proximal (α) part of the z gene account for about one-half of both the structural length and MW of the promoter z gene for β-gal.

Zamenhoff and Villarejo (Zamenhof P, Villarejo M,1972, J Bacteriol., 110, 171-178) demonstrated in *vivo* α-complementation of β-gal in 16 z gene terminator (nonsense) mutant strains of *E. coli* upon introduction of a gene fragment specifying production of a mutant Z gene polypeptide containing a small deletion in the N-terminal region of the enzyme monomer.

Since then, many sequence variants of donor and acceptor species of β-gal have been described, reviewed by Eglen (Eglen R, 2002, Assay and Drug Development Technologies, 5, 97-105). In particular, a variation developed by DiscoverX is a system for complementation of a small 4 kDa α fragment donor (ED) peptide (termed "ProLabel") with an ω deletion mutant of the enzyme acceptor (EA).

Further work reviewed by Olson and Eglen (Olson K & Eglen R, 2007, Assay and Drug Development Technologies, 5, 137-144) describes the 47-mer enzyme donor (ED) sequence (SEQ ID. No. 1):

In addition to enzyme complementation of β-Gal, complementation is a common phenomenon now reported for other proteins, including dihydrofolate reductase (Remy I & Michnick S, 2001, Proc Natl Acad Sci USA., 98, 7678-7683), β-lactamase (Wehrman T et al., 2002. Proc Natl Acad Sci USA, 99, 3469-3474), luciferase (Ozawa T., et al. 2001. Anal Chem., 73, 2516-2521) and ubiquitinase (Rojo-Niersbach E et al. 2000, Biochem J., 348, 585-590).

Henderson et al. (1986) Clinical Chemistry, 32(9), 1637-1641 (Microgenics) describes genetic engineering of β-galactosidase which led to the development of a homogeneous immunoassay system.

US5120653 (Microgenics) describes a vector comprising a DNA sequence coding for an enzyme-donor polypeptide.

US5643734 and US5604091 (Microgenics) describe methods and compositions for enzyme complementation assays for qualitative and quantitative measurement of an analyte in a sample.

PCT WO 2003/021265 (DiscoveRx) describes a genetic construct intracellular monitoring system provided for producing biologically active fusion proteins comprising a sequence encoding an enzyme donor ("ED") sequence of fused in reading frame to a sequence encoding a surrogate of a mammalian protein of interest, where the fusion protein has the function of the natural protein. Furthermore, a vector is described comprising a transcriptional and translational regulatory region functional in a mammalian host cell, a sequence encoding the ED joined to a multiple cloning site, an enzyme acceptor ("EA") protein or enzyme acceptor sequence encoding such protein that is complemented by the ED to form a functional enzyme such as β-galactosidase. Mammalian cells are employed that are modified to provide specific functions.

US7135325 (DiscoveRx) describes short enzyme donor fragments of β-galactosidase of not more than 40 amino acids.

PCT WO 2006/004936 (DiscoveRx) describes methods for determining the intracellular state of a protein as well as modifications to the protein. The method involves introducing a surrogate fusion protein comprising a member of an enzyme fragment complementation complex and a target protein. After exposing cells transformed with the surrogate fusion protein to a change in environment (e.g. a candidate drug), the cells are lysed, the lysate separated into fractions or bands by gel electrophoresis and the proteins transferred by Western blot to a membrane. The bands on the membrane are developed using the other member of the enzyme fragment complementation complex and a substrate providing a detectable signal.

US2007/0105160 (DiscoveRx) describes methods and compositions for determining intracellular translocation of proteins employing β-galactosidase fragments that independently complex to form an active enzyme. Engineered cells have two fusion constructs: one fragment bound to a protein of interest; and the other fragment bound to a compartment localizing signal. The cells are used to screen compounds for their effect on translocation, where a substrate containing high ionic strength solution is used for detection of the enzyme complex.

Verkaaf et al., (2008), Biotechnology Annual Review, 14, pages 253-274 describes G-protein-independent cell-based assays for drug discovery on GPCRs using enzyme fragment complementation (EFC) of beta-galactosidase.

### Technical Problem

Despite the existence of a number of non-radioactive assays, there is a continued need for new non-radioactive GTP binding assays for G protein-coupled receptors. These assays should be highly specific and provide a clear signal which is readily detectable over background noise. Preferably, these assays should be homogeneous in nature, obviating the requirement for separation steps and making the assays suitable for compound screening purposes, particularly high throughput drug screening.
These problems are resolved by the provision of the compounds of the present invention, which have a high binding affinity for Gα, in assays to measure ligand binding to GPCRs.

### Summary of the Invention

The present invention in its broadest sense is defined by the appended claims.

According to a first aspect of the present invention, there is provided a compound as defined in formula I: 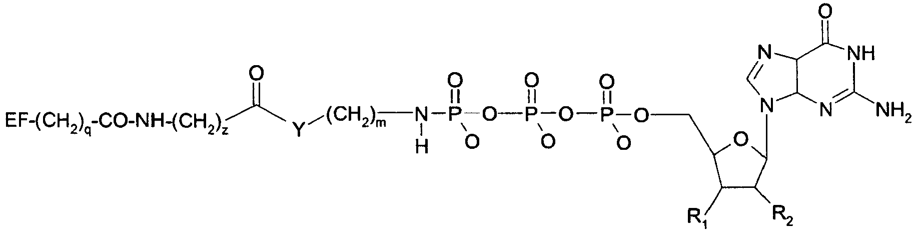 wherein:
R₁ and R₂ are independently selected from H, OH and OX, wherein X is independently selected from a group consisting of alkyl, phenyl, substituted phenyl and derivatised phenol;
m is an integer from 1 to 10;
q is an integer from 1 to 6;
z is an integer from 1 to 10;
Y is either S or NR, where R is H or alkyl; and EF is an enzyme fragment selected from the group consisting of enzyme donor (ED) and enzyme acceptor (EA); wherein EF is an enzyme fragment selected from the group of enzymes consisting of β*-*galactosidase and β-lactamase.
Compounds in accord with the invention are resistant to hydrolysis by GTPase enzymes or are "GTPase resistant".

EF is an enzyme fragment selected from the group of enzymes consisting of β-galactosidase, and β-lactamase. Preferably, EF is an enzyme donor (ED). Preferably, ED is a fragment of β-galactosidase and has the sequence shown in SEQ ID NO.1. Cys Ser Leu Ala Val Val Leu Gln Arg Arg Asp Trp Glu Asn Pro Gly Val Thr Gln Leu Asn Arg Leu Ala Ala His Pro Pro Phe Ala Ser Trp Arg Asn Ser Glu Glu Ala Arg Thr Asp Cys Pro Ser Gln Gln Leu

In one aspect, ED is a fragment of β-lactamase.

In one aspect, EF is an enzyme acceptor (EA). Preferably, EA is a fragment of β-galactosidase or a fragment of β-lactamase.

In one aspect, R₁ and R₂ are OH. In another aspect, Y is NH. In a further aspect, m is an integer from 2 to 6. In yet another aspect, q is an integer from 2 to 4. In another aspect, z is an integer from 2 to 6. Preferably, R₁ and R₂ are OH, Y is NR where R is H, m is 2, q is 2, and z is 4. More preferably, EF is an enzyme donor (ED) of β-galactosidase. Most preferably, ED has the sequence shown in SEQ. ID. NO. 1.

In another aspect, EF is an enzyme donor (ED) of β-lactamase. In a further aspect, EF is an enzyme acceptor (EA) of β-galactosidase or EF is an enzyme acceptor (EA) of β-lactamase.

In a second aspect of the present invention, there is provided a method for testing for the binding of a ligand to a G Protein Coupled Receptor (GPCR) in an enzyme complementation assay, said method comprising:
a) providing a fluid sample comprising a GPCR, a Gα subunit and a Gβγ subunit, said GPCR comprising an enzyme fragment (EF) which acts as an enzyme acceptor (EA) or an enzyme donor (ED);
b) adding a GTPase resistant compound as hereinbefore described to said fluid sample wherein said compound comprises an enzyme fragment (EF) which is capable of enzyme complementation with the enzyme fragment (EF) of said GPCR;
c) adding a ligand to the fluid sample to allow binding of said ligand to the GPCR to promote binding of the compound to said Gα subunit and thereby enzyme complementation between said enzyme donor and said enzyme acceptor to form an active enzyme;
d) adding a substrate of said active enzyme to the fluid sample; and
e) detecting a change in an optical signal resulting from the activity of the active enzyme on said substrate;
wherein the enzyme fragment (EF) is an enzyme acceptor (EA) or enzyme donor (ED) selected from the group of enzymes consisting of β-galactosidase and β-lactamase.

In another aspect, the GPCR comprises an enzyme acceptor (EA) and the GTPase resistant compound comprises an enzyme donor (ED).

Preferably, the enzyme acceptor (EA) is a fragment of β-galactosidase and the GTPase resistant compound is as hereinbefore described.

In one aspect, the enzyme fragment (EF) is attached to the carboxy terminus of the GPCR.

In one aspect, the GPCR is in the form of a membrane preparation.

In another aspect, the method is an homogeneous method. This has the advantage that no separation steps are required in the carrying out the method of the second aspect.

Preferably, the optical signal is a luminescent signal, which typically can comprise fluorescence (photoluminescence), chemiluminescence and bioluminescence.

In a third aspect of the present invention, there is provided a heterogeneous method for testing for the binding of a ligand to a G Protein Coupled Receptor (GPCR), said method comprising:
a) providing a fluid sample comprising a GPCR, a Gα subunit and a Gβγ subunit;
b) adding a GTPase resistant compound as hereinbefore described;
c) adding a ligand to the fluid sample to allow binding of said ligand to said GPCR and binding of said compound to said Gα subunit to form a complex of the GPCR and the compound;
d) separating any unassociated compound from said complex of the GPCR and the compound;
e) adding an enzyme acceptor (EA) to the complex;
f) adding a substrate of said enzyme of said enzyme acceptor to said complex of the GPCR and the compound; and
g) detecting a change in the optical signal resulting from the activity of the enzyme on said substrate;
wherein the enzyme of said enzyme acceptor is selected from the group of enzymes consisting of β-galactosidase and β-lactamase.
In one aspect, the GPCR is in the form of a membrane preparation.

Preferably, the enzyme of said enzyme acceptor is selected from the group of enzymes consisting of β-galactosidase, β-lactamase, dihydrofolate reductase, luciferase and ubiquitinase. More preferably, the enzyme is a β-galactosidase.

In one aspect, the optical signal is a luminescent signal, which typically can comprise of fluorescence (photoluminescence), chemiluminescence and bioluminescence.

### Detailed Description of the Invention

The invention is illustrated by reference to the following examples.

### 1. Synthesis of GTPase-resistant GTP Analogues

The preparation of nucleoside triphosphate analogue of formula 1: 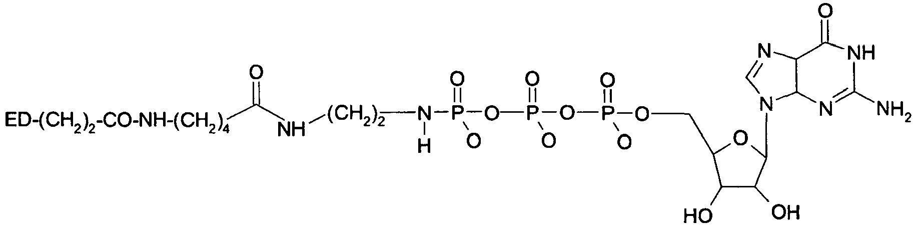 wherein ED is an enzyme donor.

### 1.1 Synthesis of O^{5'}-[3-(2-aminoethylamino)-1,2,3-trihydroxy-triphosphoryl-guanosine: Compound 1

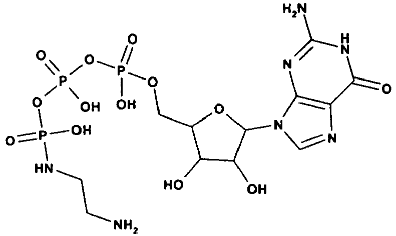

The tetra-lithium salt of GTP (25mg, 0.048mmol) and N-(3-dimethylaminopropyl)-N'-ethylcarbodiimide hydrochloride (36mg, 0.190mmol) were dissolved in 4ml of 0.1 M triethanolamine hydrochloride buffer (pH 7.2) in a 25ml round-bottomed flask fitted with a magnetic stirrer bead. N-Fmoc-ethane-1,2-diamine hydrobromide (113mg, 0.31 mmol) was suspended in 1.5ml of 1,4-dioxan and added to the stirred solution in the flask. DMF was added dropwise to the suspension in the flask until it became homogeneous. The solution was stirred at ambient temperature under an atmosphere of nitrogen for 20 hours. TLC (RP-18, 40:60 methanol:water) showed that all the GTP had reacted. The solution was evaporated to dryness under vacuum. 2.5ml of a mixture of 20:80 piperidine:DMF was added to the residue and the mixture stirred at ambient temperature for 15 minutes. The solution was then evaporated to dryness under vacuum. The residue was dissolved in water (10ml) and extracted with diethyl ether (2 x 10ml), the aqueous phase was then evaporated to dryness under vacuum. TLC (RP-18, 40:60 methanol:water) showed a single spot (R_{f} 0.75) which turned purple when sprayed with ninhydrin.

The residue was dissolved in water and purified by HPLC using a MonoQ™ 10/10 column (Amersham Biosciences) eluting with a gradient of water to 100% 0.5M triethylammonium acetate solution (pH 7.0) over 60 minutes at a flow of 3ml/minute. Detection was at 260nm. The major product eluted after 36 minutes. This material was evaporated to dryness under vacuum and the residue dissolved in a minimal volume of water. This was further purified by reverse phase HPLC using a 250 x 10 mm Jupiter™ C-18 column (Phenomenex) eluting with 0.1 M triethylammonium acetate solution (pH 7.0) at a flow of 4ml/minute. Detection was at 260nm. A single peak eluted after 11.3 minutes. This material was evaporated to dryness under vacuum, the residue was dissolved in water and the process repeated several times to remove as much triethylammonium acetate as possible to give compound (1) as a colourless solid.

Mass spectrometry (ES⁺) gave MH⁺ at 566 and MNa⁺ at 588.
(C₁₂H₂₂N₇O₁₃P₃ requires 565). Calculated yield from absorption at 253nm was 7.1mg (0.012mmol, 25%)

### 1.2 Preparation of β-galactosidase Enzyme Donor Fragment

The carboxy terminus of an appropriate β-galactosidase enzyme donor fragment is derivatised to a N-hydroxy-succinimide (NHS) ester using conventional methods and with the N-terminus reversibly protected (e.g. see methods described in R. Kluger and A. Alagic, Bioorganic Chemistry (2004), 32, 451-472). A number of β-galactosidase enzyme donor fragments are known in the scientific and patent literature (see 'Enzyme Complementation' above). For example, the 47-mer β-galactosidase enzyme donor described by Olson and Eglen (Assay and Drug Development Technologies 2007, 5, 97-105) and shown (SEQ ID No. 1) below: is derivatised in this way to yield a enzyme donor (ED)-NHS ester.

### 1.3 Coupling of β-galactosidase Enzyme Donor Fragment to 05'-[3-(2-aminoethylamino)-1,2,3-trihydroxy-triphosphoryl-guanosine

The β-galactosidase ED-NHS ester from step 1.2 above is reacted with O^{5'}-[3-(2-aminoethylamino)-1,2,3-trihydroxy-triphosphoryl-guanosine (compound 1 above) in the presence of N-(3-dimethylaminopropyl)-N'-ethylcarbodiimide hydrochloride to form the GTPase resistant GTP analogue (compound 2): 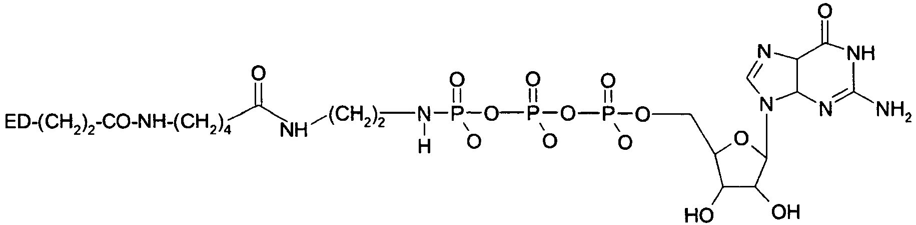 wherein ED is of the sequence as depicted in SEQ ID No.1.

### 1.4 Purification of GTPase Resistant GTP Analogue (compound 2)

Purification of the GTP-ED analogue (compound 2) is carried out by HPLC using a weak anion exchange resin such as DEAE and elution with sodium chloride or another appropriate salt gradient. Compound 2is de-salted by further HPLC purification using reverse phase resin and eluted with a gradient of triethylammonium acetate (TEAA) or bicarbonate solution into acetonitrile. After collection of the required compound, excess TEAA is removed by lyophilisation.

### 2. Production of GPCR Preparations Derivatised with an Enzyme Acceptor Fragment

The method involves creation of a polypeptide chimera comprising a GPCR which is linked to a mutant form of a β-galactosidase enzyme acceptor (EA) fragment. The latter enzyme component lacks the coding for key amino acids at chosen sites of the β-Gal gene, and the expressed protein would normally exist as an enzymatically inactive dimer.

One of the more widely studied examples of a β-Gal EA peptide is the X90-acceptor peptide that has a deletion in the last 10 amino acids (1013-1023). The X90 EA peptide exists as a monomer and can be complemented by a corresponding ED fragment of β-Gal, such as CNBr24, a cyanogen bromide digestion product of β-galactosidase consisting of amino acids 990-1023, to reform enzymatically active tetramer (Welphy et al., 1980, Biochem. Biophys. Res. Common., 93, 223).

The GPCR chimera protein is constructed, comprising a GPCR fused at the C-terminus, to an enzyme acceptor (EA) fragment. In one embodiment, the GPCR is a β2-adrenergic receptor. The cDNA (full length) sequences are available from commercial sources (e.g. Clontech).

A vector is constructed (e.g. pCl-neo vector from Promega, Cat no. E1841) using techniques well known in the art comprising the gene-of interest (GOI) coding for the chimera GPCR/enzyme acceptor (EA). The pCl-neo Mammalian Expression Vector carries the human cytomegalovirus (CMV) immediate-early enhancer/promoter region to promote constitutive expression of cloned DNA inserts in mammalian cells. This vector also contains the neomycin phosphotransferase gene, a selectable marker for mammalian cells. The pCl-neo Vector can be used for transient expression or for stable expression by selecting transfected cells with the antibiotic G-418.

Transfection of target cells (e.g. mammalian cells) using a transfection agent such as Fugene6, with the above-described vector is carried out in accordance with Manufacturer's instructions and following the principles outlined by Sambrook and Russell (Molecular Cloning, A Laboratory Manual, 3rd Edition, Volume 3, Chapter 16, Section 16.1-16.54).

The resulting transfected cells are maintained in culture or frozen for later use according to standard practices. These cells express the desired GPCR-EA chimera protein, as described above. In one embodiment, membrane preparations from these cells are prepared for further experimentation, as described below (section 3.1).

### 3. GPCR Assays

Methods of carrying out *in vitro* GPCR assays are well documented in the literature and are described, for example, in WO2006/035207 and WO2006035208 (GE Healthcare UK Limited).

By way of illustration only, and without limitation to the specific assays disclosed, the following assays are described to demonstrate different assays of utilising the compounds of the invention to test for ligand binding to a GPCR.

### 3.1 Homogeneous Assays

Intact cells expressing a GPCR, in which the carboxy-terminus of the GPCR is derivatised with the EA fragment of β-galactosidase (or in another embodiment, receptor membrane preparations from these cells) are allowed to come into contact in a tube (microwell) in the presence of a suitable buffer, with the GTP-ED adduct. In the presence of a suitable GPCR ligand (e.g. isoproterenol, noradrenaline, salmeterol,, denopamine), GTP-ED will compete with added guanosine diphosphate (GDP) in the system and will become closely associated through space with the Gα component of the G-protein trimeric complex (G_{αβγ}). In the "activated phase" of the GPCR (plus ligand), the G-protein complex is closely associated with the carboxy terminus of the GPCR. This will lead to close proximity of the GPCR-EA and the GTP-ED components which in turn will lead to β-galactosidase_enzyme complementation. Upon addition of the pro-luminescent 1,2-dioxetane substrate, a luminescent signal will be generated which can be detected in a photomultiplier device.

In the absence of a suitable GPCR ligand, GDP, rather than GTP, binds favourably to β-galactosidase. If there is any degree of endogenous activation, the majority of any Gα, with any resultant bound GTP-ED, will be dissociated through space from the remainder of the G-protein complex. This cannot then result in enzyme complementation and therefore no signal above background is seen upon addition of β-galactosidase substrate.

In this system, a signal increase arises from a higher degree of β-galactosidase complementation. This in turn, reflects a higher degree of GTP-ED recruitment to Gα, which is directly proportional to the potency of activation of the GPCR ligand.

### 3.2 Homogeneous Assay method for GPCR Activation

5000 cells per well which express the GPCR-EA protein (see section 2 above) are transferred into a 96 (or 384) well culture plate and incubated overnight at 37°C in a 5% atmosphere of CO₂. An aliquot (e.g. 5µl) of the GTP-ED compound (compound 2) is added to each well either before, simultaneously or following addition of a small volume (e.g. 5µl) of a suitable test compound or ligand (e.g. isoproterenol, noradrenaline, salmeterol, denopamine) dissolved or suspended in a non-toxic solvent and the plate incubated for 1 hour at 37°C in a 5% atmosphere of CO₂ to allow enzyme complementation to occur. An appropriate luminescent substrate of β-galactosidase (e.g. 5-acetylaminofluorescein di-b-D-galactopyranoside (X-gal) from Invitrogen; 5-lodo-3-indolyl-beta -D-galactopyranoside from Sigma; or 5-acetylaminofluorescein di-b-D-galactopyranoside from Invitrogen) is added to each well and the plate incubated for 1 hour at 37°C in a 5% CO₂ atmosphere. A change in the fluorescent signal is read using a plate reader or imager (e.g. In Cell Analyzer, GE Healthcare).

### 3.3 Heterogeneous Assay

In another embodiment (heterogeneous format), a native (non-modified) GPCR membrane preparation in a suitable buffer is allowed to come into contact in a tube or microwell, with the GTP-ED product (e.g. compound 2). In the presence of GPCR ligand, GTP-ED will be closely associated through space with the Gα component of the G-protein trimeric complex. The entire GPCR-G-protein-GTP-ED complex is then filtered and the degree of GTP-ED associated with the GPCR (via binding to Gα) is assessed by addition of a β-galactosidase substrate (e.g. pro-luminescent 1,2-dioxetane) and a β-galactosidase_EA acceptor preparation. Complementation of β-Gal occurs, leading to generation of a luminescent signal which is detected using an imaging system (e.g. IN Cell Analzyer, GE Healthcare). The higher the signal, the greater the quantity of binding of GTP-ED and this in turn, will be directly proportional to the potency of activation of the GPCR ligand.

## Claims

1. A compound as defined in formula I: 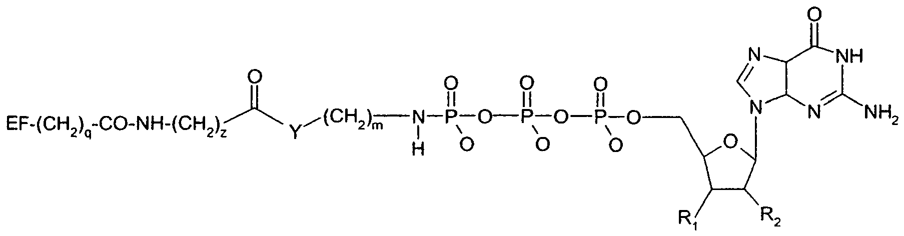 wherein:
R₁ and R₂ are independently selected from H, OH and OX, wherein X is independently selected from a group consisting of alkyl, phenyl, substituted phenyl and derivatised phenol;
m is an integer from 1 to 10;
q is an integer from 1 to 6;
z is an integer from 1 to 10;
Y is either S or NR, where R is H or alkyl;
EF is an enzyme fragment selected from the group consisting of enzyme donor (ED) and enzyme acceptor (EA);
wherein EF is an enzyme fragment selected from the group of enzymes consisting of β-galactosidase and β-lactamase.

2. The compound according to claim 1, wherein EF is an enzyme donor (ED).

3. The compound according to claim 1 or 2, wherein ED is a fragment of β-galactosidase.

4. The compound according to claim 2 or 3, wherein ED has the sequence shown in SEQ ID No. 1.

5. The compound according to claim 2, wherein ED is a fragment of β-lactamase.

6. The compound according to claim 1, wherein EF is an enzyme acceptor (EA).

7. The compound according to claim 6, wherein EA is a fragment of β-galactosidase.

8. The compound according to claim 6, wherein EA is a fragment of β-lactamase.

9. The compound according to any preceding claim wherein R₁ and R₂ are OH, Y is NR where R is H, m is 2, q is 2, and z is 4.

10. The compound according to any of claims 1 to 3 and 9, wherein EF is an enzyme donor (ED) of β-galactosidase.

11. The compound according to claim 10, wherein ED has the sequence shown in SEQ. ID. No. 1.

12. The compound according to any of claims 1, 2, 5 and 9, wherein EF is an enzyme donor (ED) of β-lactamase.

13. The compound according to any of claims 1, 6, 7 and 9, wherein EF is an enzyme acceptor (EA) of β-galactosidase.

14. The compound according to any of claims 1, 6, 8 and 9, wherein EF is an enzyme acceptor (EA) β-lactamase.

15. A method for testing for the binding of a ligand to a G Protein Coupled Receptor (GPCR) in an enzyme complementation assay, said method comprising:
a) providing a fluid sample comprising a GPCR, a Gα subunit and a Gβγ subunit, said GPCR comprising an enzyme fragment (EF) which acts as an enzyme acceptor (EA) or an enzyme donor (ED);
b) adding a GTPase resistant compound according to any of claims 1 to 14 to said fluid sample wherein said compound comprises an enzyme fragment (EF) which is capable of enzyme complementation with the enzyme fragment (EF) of said GPCR;
c) adding a ligand to the fluid sample to allow binding of said ligand to the GPCR to promote binding of the compound to said Gα subunit and thereby enzyme complementation between said enzyme donor and said enzyme acceptor to form an active enzyme;
d) adding a substrate of said active enzyme to the fluid sample; and
e) detecting a change in an optical signal resulting from the activity of the active enzyme on said substrate;
wherein the enzyme fragment (EF) is an enzyme acceptor (EA) or enzyme donor (ED) selected from the group of enzymes consisting of β-galactosidase- and β-lactamase.

16. The method according to claim 15, wherein the GPCR comprises an enzyme acceptor (EA) and the GTPase resistant compound comprises an enzyme donor (ED).

17. The method according to claim 15, wherein the enzyme acceptor (EA) is a fragment of β-galactosidase and the GTPase resistant compound is as defined in either of claims 10 or 11.

18. The method according to any of claims 15 to 17, wherein said method is an homogeneous method.

19. A heterogeneous method for testing for the binding of a ligand to a G Protein-Coupled Receptor (GPCR), said method comprising:
a) providing a fluid sample comprising a GPCR, a Gα subunit and a Gβγ subunit;
b) adding a GTPase resistant compound according to any of claims 1 to 14;
c) adding a ligand to the fluid sample to allow binding of said ligand to said GPCR and binding of said compound to said Gα subunit to form a complex of the GPCR and the compound;
d) separating any unassociated compound from said complex of the GPCR and the compound;
e) adding an enzyme acceptor (EA) to the complex;
f) adding a substrate of said enzyme of said enzyme acceptor to said complex of the GPCR and the compound; and
g) detecting a change in the optical signal resulting from the activity of the enzyme on said substrate;
wherein the enzyme of said enzyme acceptor is selected from the group of enzymes consisting of β-galactosidase and β-lactamase.

20. The method according to claim 19, wherein the enzyme is a β-galactosidase.

## Patentansprüche

1. Verbindung gemäß der Formel I: 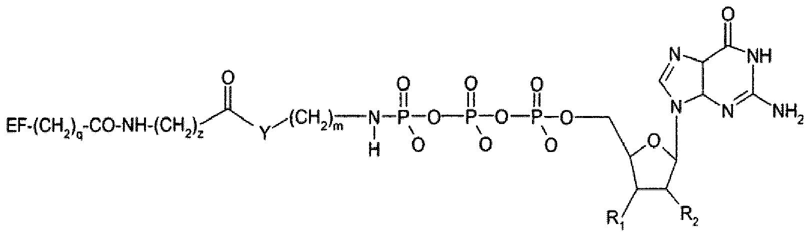 worin:
R₁ und R₂ unabhängig aus H, OH und OX gewählt sind, worin X unabhängig aus einer Gruppe gewählt ist, die sich aus Alkyl, Phenyl, substituiertem Phenyl und derivatisiertem Phenol zusammensetzt;
m für eine ganze Zahl von 1 bis 10 steht;
q für eine ganze Zahl von 1 bis 6 steht;
z für eine ganze Zahl von 1 bis 10 steht;
Y für entweder S oder NR steht, wo R für H oder Alkyl steht;
EF für ein Enzymfragment steht, das aus jener Gruppe gewählt ist, die sich aus Enzymdonor (ED) und Enzymakzeptor (EA) zusammensetzt;
wobei EF für ein Enzymfragment steht, das aus jener Gruppe von Enzymen gewählt ist, die sich aus β-Galaktosidase und β-Laktamase zusammensetzt.

2. Verbindung nach Anspruch 1, wobei EF für einen Enzymdonor (ED) steht.

3. Verbindung nach Anspruch 1 oder 2, wobei ED für ein Fragment von β-Galaktosidase steht.

4. Verbindung nach Anspruch 2 oder 3, wobei ED die in SEQ ID N° 1 dargestellte Sequenz aufweist.

5. Verbindung nach Anspruch 2, wobei ED für ein Fragment von β-Laktamase steht.

6. Verbindung nach Anspruch 1, wobei EF für einen Enzymakzeptor (EA) steht.

7. Verbindung nach Anspruch 6, wobei EA für ein Fragment von β-Galaktosidase steht.

8. Verbindung nach Anspruch 6, wobei EA für ein Fragment von β-Laktamase steht.

9. Verbindung nach einem der vorstehenden Ansprüche, wobei R₁ und R₂ für OH stehen, Y für NR steht, wo R für H steht, m für 2, q für 2 und z für 4 steht.

10. Verbindung nach einem der Ansprüche 1 bis 3 und 9, wobei EF für einen β-Galaktosidase-Enzymdonor-(ED) steht.

11. Verbindung nach Anspruch 10, wobei ED die in SEQ ID N° 1 dargestellte Sequenz aufweist.

12. Verbindung nach einem der Ansprüche 1, 2, 5 und 9, wobei EF für einen β-Laktamase-Enzymdonor-(ED) steht.

13. Verbindung nach einem der Ansprüche 1, 6, 7 und 9, wobei EF für einen β-Galaktosidase-Enzymakzeptor-(EA) steht.

14. Verbindung nach einem der Ansprüche 1, 6, 8 und 9, wobei EF für einen β-Laktamase-Enzymakzeptor-(EA) steht.

15. Verfahren zum Testen auf die Bindung eines Liganden an einen G-Protein-gekoppelten Rezeptor (GPCR) in einem Enzymkomplementationsassay, wobei das Verfahren umfasst:
a) Bereitstellen einer fluiden Probe, die einen GPCR, eine Gα-Untereinheit und eine Gβγ-Untereinheit umfasst, wobei der GPCR ein Enzymfragment (EF) umfasst, das als Enzymakzeptor (EA) oder Enzymdonor (ED) fungiert;
b) Hinzufügen einer GTPase-resistenten Verbindung nach einem der Ansprüche 1 bis 14 zu der fluiden Probe, wobei die Verbindung ein Enzymfragment (EF) umfasst, das zur Enzymkomplementation mit dem Enzymfragment (EF) des GPCR in der Lage ist;
c) Hinzufügen eines Liganden zu der fluiden Probe, um eine Bindung des Liganden an den GPCR zu ermöglichen, um eine Bindung der Verbindung an die Gα-Untereinheit und dadurch Enzymkomplementation zwischen dem Enzymdonor und dem Enzymakzeptor zu fördern, damit ein aktives Enzym gebildet wird;
d) Hinzufügen eines Substrats des aktiven Enzyms zu der fluiden Probe; und
e) Erfassen einer Veränderung eines optischen Signals, resultierend aus der Aktivität des aktiven Enzyms auf dem Substrat;
wobei das Enzymfragment (EF) ein Enzymakzeptor (EA) oder ein Enzymdonor (ED) ist, der aus jener Gruppe von Enzymen gewählt ist, die sich aus β-Galaktosidase und β-Laktamase zusammensetzt.

16. Verfahren nach Anspruch 15, wobei der GPCR einen Enzymakzeptor (EA) umfasst und die GTPase-resistente Verbindung einen Enzymdonor (ED) umfasst.

17. Verfahren nach Anspruch 15, wobei der Enzymakzeptor (EA) ein Fragment von β-Galaktosidase ist und die GTPase-resistente Verbindung wie in einem der Ansprüche 10 und 11 definiert ist.

18. Verfahren nach einem der Ansprüche 15 bis 17, wobei das Verfahren ein homogenes Verfahren ist.

19. Heterogenes Verfahren zum Testen auf die Bindung eines Liganden an einen G-Protein-gekoppelten Rezeptor (GPCR), wobei das Verfahren umfasst:
a) Bereitstellen einer fluiden Probe, die einen GPCR, eine Gα-Untereinheit und eine Gβγ-Untereinheit umfasst;
b) Hinzufügen einer GTPase-resistenten Verbindung nach einem der Ansprüche 1 bis 14;
c) Hinzufügen eines Liganden zu der fluiden Probe, um eine Bindung des Liganden an den GPCR und eine Bindung der Verbindung an die Gα-Untereinheit zu ermöglichen, damit ein Komplex aus dem GPCR und der Verbindung gebildet wird;
d) Trennen jeglicher nicht assoziierter Verbindung von dem Komplex aus dem GPCR und der Verbindung;
e) Hinzufügen eines Enzymakzeptors (EA) zu dem Komplex;
f) Hinzufügen eines Substrats des Enzyms des Enzymakzeptors zu dem Komplex aus dem GPCR und der Verbindung; und
g) Erfassen einer Veränderung des optischen Signals, resultierend aus der Aktivität des Enzyms auf dem Substrat;
wobei das Enzym des Enzymakzeptors aus jener Gruppe von Enzymen gewählt ist, die sich aus β-Galaktosidase und β-Laktamase zusammensetzt.

20. Verfahren nach Anspruch 19, wobei das Enzym eine β-Galaktosidase ist.

## Revendications

1. Composé selon la formula I: 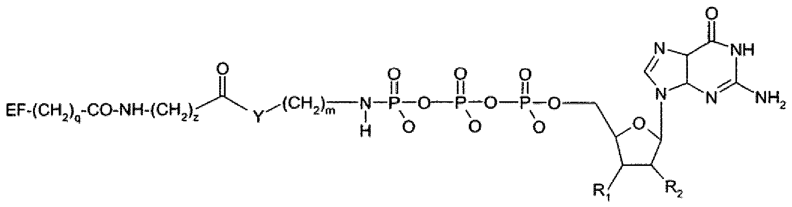 dans laquelle :
R₁ et R₂ sont sélectionnés indépendamment parmi H, OH et OX, où X est sélectionné indépendamment parmi un groupe constitué par un alkyle, un phényle, un phényle substitué et un phénol dérivé ;
m est un entier de 1 à 10 ;
q est un entier de 1 à 6 ;
z est un entier de 1 à 10 ;
Y représente soit S soit NR, où R représente H ou un alkyle,
EF est un fragment d'enzyme sélectionné parmi le groupe constitué par un donneur d'enzyme (ED) et un receveur d'enzyme (EA) ;
dans lequel EF est un fragment d'enzyme sélectionné parmi le groupe d'enzymes constitué par la β-galactosidase et la β-lactamase.

2. Composé selon la revendication 1, dans lequel EF est un donneur d'enzyme (ED).

3. Composé selon la revendication 1 ou 2, dans lequel ED est un fragment de β-galactosidase.

4. Composé selon la revendication 2 ou 3, dans lequel ED présente la séquence représentée par la séquence SEQ ID N °1.

5. Composé selon la revendication 2, dans lequel ED est un fragment de β-lactamase.

6. Composé selon la revendication 1, dans lequel EF est un receveur d'enzyme (EA).

7. Composé selon la revendication 6, dans lequel EA est un fragment de β-galactosidase.

8. Composé selon la revendication 6, dans lequel EA est un fragment de β-lactamase.

9. Composé selon l'une quelconque des revendications précédentes dans lequel R₁ et R₂ représentent OH, Y représente NR où R représente H, m est égal à 2, q est égal à 2, et z est égal à 4.

10. Composé selon l'une quelconque des revendications 1 à 3 et 9, dans lequel EF est un donneur d'enzyme (ED) de β-galactosidase.

11. Composé selon la revendication 10, dans lequel ED présente la séquence représentée par la séquence SEQ. ID. N °1.

12. Composé selon l'une quelconque des revendications 1, 2, 5 et 9, dans lequel EF est un donneur d'enzyme (ED) de β-lactamase.

13. Composé selon l'une quelconque des revendications 1, 6, 7 et 9, dans lequel EF est un receveur d'enzyme (EA) de β-galactosidase.

14. Composé selon l'une quelconque des revendications 1, 6, 8 et 9, dans lequel EF est un receveur d'enzyme (EA) de β-lactamase.

15. Procédé d'analyse de la liaison d'un ligand à un récepteur couplé aux protéines G (GPCR) dans un dosage de complémentation d'enzyme, ledit procédé comprenant :
a) la préparation d'un échantillon fluide comprenant un GPCR, une sous-unité Ga et une sous-unité Gby, ledit GPCR comprenant un fragment d'enzyme (EF) qui agit en tant que receveur d'enzyme (EA) ou donneur d'enzyme (ED) ;
b) l'ajout d'un composé résistant à la GTPase selon l'une quelconque des revendications 1 à 14 audit échantillon fluide dans lequel ledit composé comprend un fragment d'enzyme (EF) qui peut assurer la complémentation d'enzyme avec le fragment d'enzyme (EF) dudit GPCR;
c) l'ajout d'un ligand à l'échantillon fluide afin de permettre la liaison dudit ligand au GPCR de manière à favoriser la liaison du composé à la ladite sous-unité Gα et ainsi, la complémentation d'enzyme entre ledit donneur d'enzyme et ledit receveur d'enzyme afin de former une enzyme active ;
d) l'ajout d'un substrat de ladite enzyme active à l'échantillon fluide ; et
e) la détection d'un changement sur un signal optique résultant de l'activité de l'enzyme active sur ledit substrat ;
dans lequel le fragment d'enzyme (EF) est un receveur d'enzyme (EA) ou donneur d'enzyme (ED) sélectionné parmi le groupe d'enzymes constitué par la β-galactosidase et la β-lactamase.

16. Procédé selon la revendication 15, dans lequel le GPCR comprend un receveur d'enzyme (EA) et le composé résistant à la GTPase comprend un donneur d'enzyme (ED).

17. Procédé selon la revendication 15, dans lequel le receveur d'enzyme (EA) est un fragment de β-galactosidase et le composé résistant à la GTPase est selon l'une ou l'autre des revendications 10 ou 11.

18. Procédé selon l'une quelconque des revendications 15 à 17, dans lequel ledit procédé est un procédé homogène.

19. Procédé hétérogène d'analyse de la liaison d'un ligand à un récepteur couplé aux protéines G (GPCR), ledit procédé comprenant :
a) la préparation d'un échantillon fluide comprenant un GPCR, une sous-unité Gα et une sous-unité Gβγ ;
b) l'ajout d'un composé résistant à la GTPase selon l'une quelconque des revendications 1 à 14 ;
c) l'ajout d'un ligand à l'échantillon fluide afin de permettre la liaison dudit ligand audit GPCR et la liaison dudit composé à la ladite sous-unité Gα afin de former un complexe du GPCR et du composé ;
d) la séparation de tout composé non associé par rapport audit complexe du GPCR et du composé ;
e) l'ajout d'un receveur d'enzyme (EA) au complexe ;
f) l'ajout d'un substrat de ladite enzyme dudit receveur d'enzyme audit complexe du GPCR et du composé ; et
g) la détection d'un changement sur le signal optique résultant de l'activité de l'enzyme sur ledit substrat ;
dans lequel l'enzyme dudit receveur d'enzyme est sélectionnée parmi le groupe d'enzymes constitué par la β-galactosidase et la β-lactamase.

20. Procédé selon la revendication 19, dans lequel l'enzyme est la β-galactosidase.


### SEQUENCE LISTING

<110> GE Healthcare UK Limited
<120> Methods and Compounds for Testing Binding of a Ligand to a G Protein- Coupled Receptor
<130> PA0878
<160> 1
<170> Patent In version 3.2
<210> 1
   <211> 47
   <212> PRT
   <213> Artificial
<220>
   <223> B-galactosidase sequence
<400> 1
   Cys Ser Leu Ala Val Val Leu Gln Arg Arg Asp Trp Glu Asn Pro Gly
   1               5                   10                  15
   Val Thr Gln Leu Asn Arg Leu Ala Ala His Pro Pro Phe Ala Ser Trp
               20                  25                  30
   Arg Asn Ser Glu Glu Ala Arg Thr Asp Cys Pro Ser Gln Gln Leu
           35                  40                  45